(19) Europäisches Patentamt / European Patent Office / Office européen des brevets

(11) **EP 2 783 709 A1**

(12) **EUROPEAN PATENT APPLICATION**
published in accordance with Art. 153(4) EPC

(43) Date of publication:
01.10.2014 Bulletin 2014/40

(51) Int Cl.:
**A61L 31/00** *(2006.01)*

(21) Application number: **12851264.7**

(86) International application number:
**PCT/JP2012/080172**

(22) Date of filing: **21.11.2012**

(87) International publication number:
**WO 2013/077357 (30.05.2013 Gazette 2013/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.11.2011 JP 2011257174**

(71) Applicant: **Nihon Pharmaceutical Co., Ltd. Tokyo 101-0031 (JP)**

(72) Inventor: **NISHIOKA, Ayumu Izumisano-shi Osaka 598-8558 (JP)**

(74) Representative: **Von Kreisler Selting Werner - Partnerschaft von Patentanwälten und Rechtsanwälten mbB Deichmannhaus am Dom Bahnhofsvorplatz 1 50667 Köln (DE)**

(54) **SUBMUCOSAL CUSHIONING AGENT**

(57) In order to improve the resection efficiency, operability, and safety of endoscopic mucosal resection operations, a method has conventionally been employed wherein a cushion agent such as physiological saline is injected into the layer below the area designated for resection, causing the lesion to lift and protrude (hereafter simply referred to as "protrusion"), after which the lesion is removed. It is difficult to accurately remove the intended area using conventional cushion agents because applying pressure to the protrusion tends to cause deformation thereof, the degree of protrusion is low, and the cushion agent diffuses immediately after injection into the peripheral tissue, thereby causing the protrusion to disappear. The present invention solves these problems by employing a polysaccharide having a pseudoplastic viscosity such as xantham gum, carrageenan, gellan gum, guar gum, locust bean gum, sacran, or the like as a cushion agent.

EP 2 783 709 A1

**Description**

TECHNICAL FIELD

The present invention relates to a composition for submucosal cushion agent to be submucosally injected to lift a mucosal tissue of the digestive tract or the like during endoscopic resection of the mucosal tissue. More specifically, the present invention relates to the use of a polysaccharide having pseudoplastic flow properties, such as xanthan gum, as a material.

BACKGROUND ART

Endoscopic mucosal resection is clinically performed, in which a lesion site, such as a polyp or a small cancer, formed on the mucosa of the digestive tract such as the stomach or intestines is endoscopically resected without performing a laparotomy. In this case, a mucosal lesion site does not clearly protrude and a mucosal surface is slippery, which makes it reasonably technically difficult to resect the lesion site by remote control while endoscopically observing the lesion site and may cause a serious risk such as incomplete resection of the lesion site, hemorrhage, or perforation.
In order to improve the resection efficiency, operability, and safety of endoscopic mucosal resection, normal saline is conventionally injected under a lesion site to be resected to lift or protrude (hereinafter, simply referred to as lift) and then resect the lesion site. However, in this case, the lifted site is easily deformed by the application of pressure during resection, and the degree of lift is low and the lift disappears right after injection due to diffusion of the normal saline into surrounding tissue, which makes it difficult to reliably resect the site to be removed.
Further, hypertonic saline, a 50% glucose solution, or the like is sometimes used to maintain a lifted site for a longer period of time. However, the hypertonic solution is not very effective in maintaining a lift, and in addition, injection of the hypertonic solution to tissue may be injurious to the tissue. Further, such an injection composition has a problem of safety when injected into the large intestine having a thin mucosa, because there is a risk that the large intestine is perforated with a radio knife or the like when the degree of lift produced by the injection composition is low.
In order to solve such a problem, a preparation containing low-molecular sodium hyaluronate having an average molecular weight of about 800,000 has been developed. Then, an endoscopic injection composition containing high-molecular hyaluronic acid having an average molecular weight of 1,500,000 to 3,000,000 or a salt thereof has also been developed. A method using the endoscopic injection composition improves the degree and duration of lift or elevation of a lesion site as compared to the conventional methods. However, sodium hyaluronate is expensive and very viscous, and therefore the endoscopic injection composition still has a problem in handleability, because a high injection pressure is required to inject submucosally the endoscopic injection composition with an endoscopic injection needle, which affects treatment that should be performed with very high accuracy in a medical setting by medical personnel.
Further, an endoscopic local injection composition containing 1.5 to 3.5 w/v% carboxymethylcellulose sodium has also been developed. Usually, endoscopic injection needles having a needle outer diameter of 21 gauge or more (a larger gauge means a smaller actual outer diameter) and an effective tube length of 1000 mm or more are clinically used, but this endoscopic local injection composition is too viscous, and therefore it is difficult to inject submucosally the endoscopic local injection composition with a standard endoscopic injection needle having a needle diameter of 21 gauge and a tube length of 1000 mm or more.
Therefore, there has been a demand for development of a submucosal cushion agent composition having advantages of being cheaper, safer, and injectable even at a low injection pressure and producing long-lasting lift without diffusing into surrounding tissue when injected into a lesion site.

PRIOR ART DOCUMENTS

PATENT DOCUMENTS

    Patent Document 1: Japanese Patent Application No. 2000-37240
    Patent Document 2: Japanese Patent No. 4176475
    Patent Document 3: Japanese Patent No. 4761921

NON-PATENT DOCUMENTS

Non-Patent Document 1: Gastrointest. Endosc. 50(5), 701-704, 1999
Non-Patent Document 2: Gastrointest. Endosc. 50(2), 251-256, 1999

## SUMMARY OF THE INVENTION

### PROBLEMS TO BE SOLVED BY THE INVENTION

Therefore an object of the present invention is to provide an excellent cushion agent composition that is highly safe, produces long-lasting lift without diffusing even when injected into a lesion site, and is injectable at a low injection pressure but again increases in viscosity in an injection site.

### SOLUTIONS TO THE PROBLEMS

In order to obtain a submucosal cushion agent composition that can satisfy the above requirements, the present inventors have intensively studied, and as a result, have found that a cushion agent composition satisfying the above requirements can be obtained by using a polysaccharide having pseudoplastic viscosity such as xanthan gum, carrageenan, gellan gum, guar gum, locust bean gum, or sacran. The present inventors have further intensively studied and completed the present invention.

More specifically, the present invention provides the following:

(1) a composition for submucosal cushion agent including, as an active ingredient, a polysaccharide having pseudoplastic viscosity;

(2) the composition for submucosal cushion agent according to the above (1), wherein the polysaccharide having pseudoplastic viscosity is xanthan gum, carrageenan, gellan gum, guar gum, locust bean gum, sacran, or a salt thereof;

(3) the composition for submucosal cushion agent according to the above (1) or (2), wherein the polysaccharide having pseudoplastic viscosity is xanthan gum or a salt thereof;

(4) the composition for submucosal cushion agent according to any one of the above (1) to (3), wherein the polysaccharide has a concentration of 0.05 to 3.5 w/v%;

5) the composition for submucosal cushion agent according to any one of the above (1) to (4), wherein an injection pressure of the composition when pushed out of an endoscopic injection needle having a needle diameter of 23 G connected to a tube with an effective length of 1600 mm measured by a texture analyzer (EZ Test 500 N: manufactured by SHIMADZU CORPORATION) at a constant rate of 100 mm/min is 0.1 to 12 kgf.

(6) a method of forming a submucosal cushion agent comprising injecting a polysaccharide having pseudoplastic viscosity described in any one of the above (1) to (5) in a submucosa of a subject.

The term "pseudoplastic" is used as follows: for example, "a fluid in which an apparent viscosity or consistency decreases with an increase in shear rate" is referred to as a pseudoplastic fluid in this specification. That is, when a force such as a shear force is applied to an aqueous solution of the saccharide, the molecules of the sacchride are disentangled and aligned so that the aqueous saccharide solution decreases in viscosity, but when the aqueous sacchride solution is returned to a resting state, the molecules of the sugar are again entangled together so that the aqueous sugar solution recovers its initial viscosity. Such a decrease in the viscosity of the fluid is not time-dependent, and the fluid instantaneously decreases in viscosity by the application of pressure at the time when the fluid is injected to the tissue with the endoscopic injection needle but quickly recovers its initial viscosity just after release from the pressure.

Pseudoplasticity can be evaluated using a Casson yield value. In the present invention, a higher Casson yield value means that viscosity at rest is higher and therefore lift can be maintained for a longer period of time without diffusion. A too high Casson yield value causes a high injection pressure, which affects handleability during injection. The Casson yield value of the cushion agent composition according to the present invention is usually in the range of 0.1 to 100, preferably 0.5 to 75, more preferably 1 to 50.

Examples of a material having pseudoplasticity include polysaccharides such as xanthan gum, carrageenan, gellan gum, guar gum, locust bean gum, and sacran. Among them, xanthan gum is preferred.

Further, these materials having pseudoplasticity can be used in combination of two or more of them. For example, xanthan gum and locust bean gum or xanthan gum and guar gum can be used in combination.

Xanthan gum is a polysaccharide produced by bacteria called Xanthomonas campestris and composed of sugars such as mannose, glucose, and glucuronic acid. The main chain of xanthan gum is composed of $\beta$-1,4-linked glucose residues, and a side chain containing two molecules of mannose and glucuronic acid is linked to every other glucose residue on the main chain. The C-6 position of mannose linked to the main chain is frequently acetylated, and therefore terminal mannose in the side chain is pyruvated. Xanthan gum is a polysaccharide wherein a ratio of side chain to main chain is high and which is highly negatively charged by carboxyl groups and pyruvic acid contained in the side chains.

Carrageenan is one of linear sulfur-containing polysaccharides and is an anionic polymer compound composed of D-galactose (or 3,6-anhydro-D-galactose) and sulfuric acid. Carrageenan is also called "carrageenin", and its CAS registry number is 9000-07-1. Carrageenan is usually obtained from red algae by alkali extraction. Carrageenan is similar in composition to agarose (which is a major component of agar) that is also obtained from red algae, but is different from agarose in that its sulfuric acid content is larger. Carrageenan is an elastic polymer and forms double helix structures, and the double helix structures are held together to form a gel at room temperature. Carrageenan is used as a gelling agent, a thickening agent, or a stabilizer in industrial applications such as food industry.

Gellan gum is a polysaccharide extracellularly produced by bacteria called Sphingomonas elodea, and is widely used in various foods as a thickening stabilizer (thickening, gelling, or stabilizing agent). Gellan gum is divided into two types: deacylated gellan gum and native gellan gum. Gellan gum is a linear heteropolysaccharide wherein a repeating unit is composed of four sugars; glucose, glucuronic acid, glucose, and rhamnose and has a carboxyl group derived from glucuronic acid. The difference between deacylated gellan gum and native gellan gum is the presence or absence of an acetyl group and a glyceryl group in 1-3-linked glucose. Deacylated gellan gum (trade name: KELCOGEL) is obtained by removing these acetyl and glyceryl groups. These two types of gellan gum are very different in physical properties depending on the presence or absence of an acyl group. Native gellan gum is characterized by, for example, forming an elastic gel with little syneresis like a rice cake and having a high gelling temperature, freezing/thawing resistance, and the effect of dispersing insoluble solid matter when used at a low concentration. On the other hand, deacylated gellan gum is characterized by forming a highly transparent gel with good flavor release and brittle texture and having heat resistance and acid resistance.

Guar gum is a water-soluble natural polysaccharide obtained from the albumen of cluster beans. The CAS registry number of guar gum is 9000-30-0. Guar gum is a polysaccharide in which one molecule of galactose is linked as a side chain to two molecules of mannose linearly linked. Guar gum has a molecular weight of 200,000 to 300,000.

Locust bean gum is a polysaccharide extracted from the albumen of seeds of Ceratonia siliqua. Locust bean gum mainly contains galactomannan composed of β-D-mannose and α-D-galactose. The main chain of galactomannan consists of 1,4-linked β-D-mannose and the side chain consists of 1,6-linked α-D-galactose. The ratio of D-galactose to D-mannose (D-mannose : D-galactose) is about 4 : 1. Locust bean gum has a molecular weight of about 310,000.

Sacran is an agar-like material secreted into the extracellular matrix of Aphanothece sacrum that is a freshwater photosynthetic bacterium of Japanese origin. The agar-like material was analyzed by a researcher of Kaneko Laboratory in Japan Advanced Institute of Science and Technology in 2006, found to be a giant polysaccharide containing sulfated muramic acid and having an average molecular weight of 16,000,000 and a molecular chain length of 10 μm, and named "Sacran". The aqueous solution of sacran has moisture-holding ability about ten-times higher than that of sodium hyaluronate and high static viscosity, but on the other hand, has pseudoplastic properties similar to those of xanthan gum, that is, decreases in viscosity with an increase in shear rate but again rapidly increases in viscosity by a return to a resting state.

Xanthan gum used in the present invention may be obtained from any source, but is generally produced by Xanthomonas campestris using the starch of corn or the like as a raw material. The molecular weight of xanthan gum is usually 100,000 to 50,000,000, preferably 200,000 to 20,000,000, particularly preferably 1,000,000 to 10,000,000. Xanthan gum has a repeating unit composed of two molecules of glucose, two molecules of mannose, and glucuronic acid. Xanthan gum includes a salt thereof such as a potassium salt, a sodium salt, or a calcium salt. Xanthan gum turns into a viscous solution when mixed with water, and is therefore used in various applications as a thickening agent or a thickening stabilizer.

When xanthan gum is mixed with a 1% potassium chloride solution to prepare a sample solution having a xanthan gum concentration of 1%, the sample solution has a viscosity of 600 mPa·s or more, preferably 800 to 1600 mPa·s, more preferably 1200 to 1600 mPa·s as measured using a Brookfield rotational viscometer (No. 3 roter) at a rotational speed of 60 rpm at 25 ± 0.5°C. Further, when the viscosity of the sample solution adjusted to 65 ± 0.5°C is measured in the same manner as described above, V1/V2 is 1.02 to 1.45, wherein V1 is the viscosity at 25°C and V2 is the viscosity at 65°C. Examples of xanthan gum used in the present invention include KELTROL Series commercially available from Sansho Co., Ltd. such as KELTROL CG, KELTROL CG-T, and KELTROL CG-SFT, ECHO GUM Series commercially available from DSP GOKYO FOOD & CHEMICAL Co., Ltd. such as ECHO GUM T and ECHO GUM F, and SAN-ACE Series commercially available from San-Ei Gen F.F.I., Inc. such as SAN-ACE NXG-S. Among them, KELTROL Series is preferred, and KELTROL CG-T is particularly preferred.

The concentration of the polysaccharide used in the present invention is usually 0.05 to 3.5 w/v%, preferably 0.1 to 3.0 w/v%, more preferably 0.2 to 2.5 w/v% with respect to the total volume of the preparation. It is to be noted that "%" used as a unit of the concentration of the polysaccharide is w/v% unless otherwise specified.

The viscous polysaccharide used in the present invention can be diluted with an aqueous solvent used for pharmaceutical drugs, such as purified water, distilled water for injection, or pharmaceutical saline, for viscosity adjustment.

An endoscopic injection needle (endoscopic puncture needle) will be described below.

The needle diameter (G) of an endoscopic injection needle is standardized in terms of needle outer diameter, and a larger gauge number means a smaller needle outer diameter. The gauge number of an endoscopic injection needle to be used is selected depending on the site of surgery, but is usually 21 to 25 G. Even when having the same gauge number, endoscopic injection needles produced by different makers are different in inner diameter. In recent years, endoscopic injection needles designed to minimize injection pressure are commercially available. The preparation according to the present invention can be injected by an operator without difficulty even when an endoscopic injection needle having a needle diameter of 23 G or more is used. The effective tube length of an endoscopic injection needle to be used is 1000 mm or more, preferably 1500 to 2500 mm.

Method for measuring injection pressure of cushion agent composition

A 5 mL-lure lock plastic syringe is filled with a measurement solution. An endoscopic injection needle having a needle diameter of 23G and an effective tube length of 1600 mm is connected to the syringe. The syringe is fixed to a texture analyzer (EZ Test 500N manufactured by SHIMADZU CORPORATION), and the piston of the syringe is pushed at a constant rate of 100 mm/min. A force required to discharge the measurement solution in the syringe through the needle tip of the endoscopic injection needle is measured at 25°C and defined as injection pressure.

If the injection pressure is 14 kgf or more, the measurement solution is discharged through the needle tip of the endoscopic injection needle but is leaked from around the piston of the syringe, and the piston of the syringe is not moved even by pushing with the hand instead of with the texture analyzer. When the injection pressure is about 11 kgf, the measurement solution can be discharged through the needle tip when the piston of the syringe is pushed with the hand instead of with the texture analyzer.

The injection pressure of the cushion agent agent composition as measured by the above method is preferably 0.1 to 12 kgf, more preferably 0.25 to 10 kgf, even more preferably 0.5 to 10 kgf, particularly preferably 1.0 to 7 kgf.

Measurement of viscosity of injection composition

The viscosity of the injection composition is measured at a liquid temperature of $25 \pm 1°C$ using a Viscomate vibration-type viscometer (e.g., VISCOMATE VM-1G manufactured by YAMAICHI ELECTRONICS CO., LTD.).

Pseudoplastic viscosity and Casson yield value

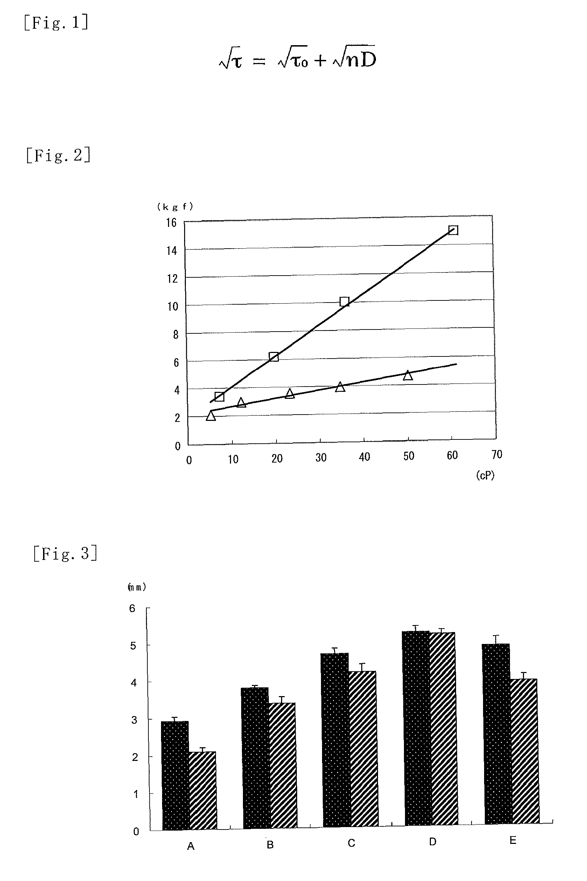

The viscosity of the injection composition is measured using a digital viscometer (e.g., Model: LVDV-II + Pro) manufactured by Brookfield Engineering Laboratories Inc. at $25 \pm 1°C$ at predetermined different rotational speeds. The Casson yield value of the injection composition is determined by a calculation formula shown in Fig. 1.

A method for producing the composition for submucosal cushion agent includes a dissolution step, a filtration-filling step, and a sterilization step.

The step of dissolving a polysaccharide thickener can be performed using a commonly-used impeller-type stirrer or dispersion-type disperser. The polysaccharide thickener may be warmed or cooled, or the head space of a tank may be purged with nitrogen or another gas according to the properties of the polysaccharide thickener. Raw materials (solid) may be charged into the same tank one after another and dissolved in water with stirring. However, a polysaccharide thickener solution and a pH adjusting agent solution are preferably prepared separately from each other and mixed. Polysaccharide thickeners are often gelled (or become cloudy) at pH outside an optimum pH range. For example, in the case of xanthan gum, a clouding phenomenon occurs on the basic side (e.g., at pH 10 or higher), and therefore an acid is previously added to a basic additive to prepare a liquid adjusted to be neutral to weakly basic (e.g., pH 6 to 8), and the liquid is added to a xanthan gum solution. The filtration filling step is not particularly limited, and filtration and filling can be performed by an ordinary method. The sterilization step is not particularly limited, either. The sterilization step may be performed by, for example, filtration sterilization, high-pressure steam sterilization, electron beam sterilization, or gamma ray sterilization, but is preferably performed by high-pressure steam sterilization. A sterilization temperature is not particularly limited, but sterilization is generally performed under conditions of 115 to 118°C for 30 minutes, 121 to 124°C for 15 minutes, or 126 to 129°C for 10 minutes.

If necessary, other pharmaceutical active ingredient, osmotic pressure adjuster, pH adjusting agent, stabilizer, coloring agent, and hemostatic may be appropriately added.

Examples of the osmotic pressure adjuster include a sugar, a sugar alcohol and a salt. Examples of the sugar include glucose and maltose. Examples of the sugar alcohol include glycerin, sorbitol, mannitol and xylitol, and sorbitol is particularly preferred. Examples of the salt include sodium chloride and the like.

The amount of the osmotic pressure adjuster to be added varies depending on the type of osmotic pressure adjuster used. When the osmotic pressure adjuster is a sugar alcohol, the amount of the sugar alcohol to be added is selected from the range of, for example, 0.1 to 6.0 w/v%. When the osmotic pressure adjuster is sorbitol, the amount of sorbitol to be added is selected from the range of, for example, 0.1 to 6.0 w/v%.

Examples of the pH adjusting agent include Tris-HCl buffer, citrate buffer and phosphate buffer. Each of them can be added in a sufficent quantity (abbreviated as "s.q."). The pH of the composition according to the present invention is preferably in the range of 4 to 10, more preferably in the range of 5 to 8.

Examples of the stabilizer include edetate sodium and the like. Each of them can be added in its appropriate amount.

Examples of the coloring agent include a pharmaceutically-acceptable dye component such as indigocarmine and the like. Each of them can be added in its appropriate amount. Addition of such a coloring agent makes it easy to visually identify an area where the preparation has been injected.

Examples of the hemostatic include components having a hemostaticwasoconstrictive effect such as epinephrine and the like. Each of them can be added in its appropriate amount. Addition of the hemostatic makes it possible to control hemorrhage during lesion resection.

The composition according to the present invention can be charged and stored in a container such as a syringe, a vial, or an ampule. The present invention may provide a set of a container filled with the composition and an endoscopic injection needle.

The composition according to the present invention is stable even after long-term storage, but pH adjustment makes the composition more stable. For example, the viscosity of the preparation is kept and the injection pressure of the preparation is not increased even after storage at 60°C for 3 weeks.

The composition according to the present invention is applied to human mucosa or surrounding tissue thereof desired to be lifted by the composition, such as submucosa, mucosa, or epithelium. Among them, submucosa is preferred. Examples of a site to which the composition according to the present invention is applied include the mucosa of digestive organs such as the esophagus, stomach, duodenum, bile duct, small intestine, large intestine, colon, and rectum, the mucosa of respiratory organs such as the lung, and the mucosa of genitourinary organs such as the urinary bladder, urethra, vagina, and uterus. Among them, the mucosa of the upper digestive tract (from the esophagus to the stomach or duodenum) and the mucosa of the lower digestive tract (the small intestine, jejunum, ileum lower than the duodenum), and large intestine (colon, rectum) are preferred.

The composition according to the present invention is also suitable for use in mucosal resection. Various techniques of mucosal resection are known which are different in a mucosal site to be resected or a tool or method used for mucosal resection. Specific examples of the mucosal resection techniques include endoscopic mucosal resection (EMR), endoscopic submucosal dissection (ESD), laparoscopic mucosal resection, uteroscopic mucosal resection, transurethral resection of bladder tumor, and laser mucosectomy. The composition according to the present invention can be used for any of these mucosal resection techniques.

The composition according to the present invention can be used in combination with a smooth muscle peristalsis inhibitor directly and locally sprayed to the inside of the digestive tract through a sprayer or an endoscope forceps during, for example, digestive tract surgery by laparotomy, endoscopic surgery, digestive tract endoscopic examination, or another medical practice in which peristalsis of the digestive tract needs to be suppressed.

EFFECTS OF THE INVENTION

According to the present invention, it is possible to provide a cushion agent composition to be submucosally injected to lift mucosal tissue of the digestive tract during endoscopic resection of the mucosal tissue. The cushion agent composition uses a polysaccharide having pseudoplastic flow properties. This makes it possible to reduce the injection pressure, at which the injection composition is injected through an endoscopic injection needle, and thereby to easily inject the injection composition and also makes it possible to maintain lift for a long time after injection.

BRIEF DESCRIPTION OF THE DRAWINGS

Fig. 1 shows a formula for calculating a Casson yield value, which represents a relationship established between shear stress and shear rate in a fluid containing a dispersed substance.

Fig. 2 is a graph showing the results of measuring the injection pressure and viscosity of each of injection solutions and comparative solutions, wherein the vertical axis represents the injection pressure (kfg) and the horizontal axis represents the viscosity (cP).

Fig. 3 is a graph showing the results of measuring the thickness of gastric submucosa (mm) just after (left-side bar of each pair of bars) and 30 minutes after (right-side bar of each pair of bars) injection of each of injection compositions, phosphate buffered saline (PBS), and a 0.4 w/v% sodium hyaluronate solution, wherein the vertical axis represents the thickness of gastric submucosa (mm) and the letters A to E along the horizontal axis correspond to A:phosphate buffered saline (PBS), B:xanthan gum 0.25 w/v% (0.25% XA), C:xanthan gum 0.50 w/v% (0.50% XA), D:xanthan gum 1.0 w/v% (1.0% XA), and sodium hyaluronate 0.4 w/v% (0.4% HA), respectively.

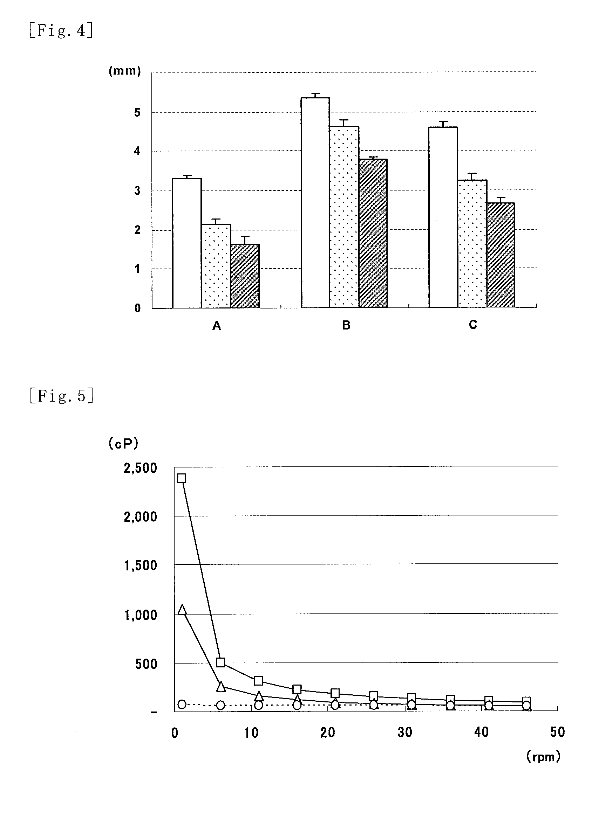

Fig. 4 is a graph showing the results of measuring the thickness of gastric submucosa (mm) just after (left-side bar of each group of bars), 1 hour after (middle bar of each group of bars), and 4 hours after (right-side bar of each group of bars) injection of each of injection compositions, normal saline, and a 0.4 w/v% sodium hyaluronate solution, wherein the vertical axis represents the thickness of gastric submucosa (mm) and the letters A to C along the horizontal axis correspond to A: normal saline, B: xanthan gum 0.50 w/v% (0.50% XA), and C: sodium hyaluronate 0.4 w/v% (0.4% HA), respectively.

Fig. 5 is a graph showing the results of measuring the viscosity of each of injection compositions and a comparative solution at 25°C at different rotational speeds while the rotational speed was increased from an initial rotational speed of 1 rpm in increments of every 5 rpm, wherein the vertical axis represents the viscosity (cP) and the horizontal axis represents the rotational speed (rpm).

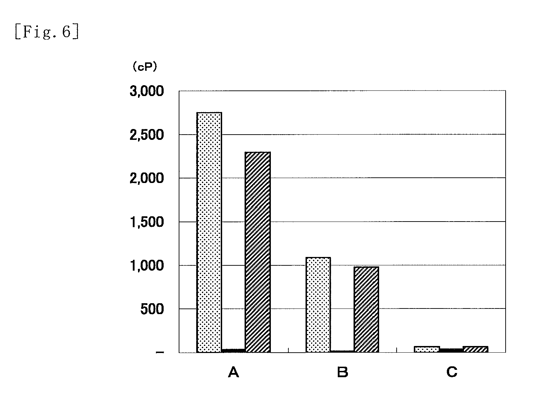

Fig. 6 is a graph showing the results of measuring the viscosity of each of injection compositions and a comparative solution at an initial rotational speed of 1 rpm (left-side bar of each group of bars), then at 200 rpm (middle bar of each group of bars) and then at 1 rpm (right-side bar of each group of bars), wherein the vertical axis represents the viscosity (cP) and the letters A to C along the horizontal axis correspond to A: xanthan gum 1.0 w/v% (1.0% XA), B: xanthan gum 0.5 w/v% (0.5% XA), and C: sodium hyaluronate 0.4 w/v% (0.4% HA), respectively.

EMBODIMENTS OF THE INVENTION

Hereinbelow, the present invention will be described more specifically with reference to the following examples, comparative examples, and test examples.

Example 1

The injection pressure of each of the following injection compositions was measured by the method described in [0016]. It is to be noted that the measurement was performed using the following two kinds of endoscopic injection needles. Further, each of the injection compositions was actually injected manually to determine the degree of difficulty of injection.

Endoscopic injection needle 1: Endoscopic puncture needle with a needle diameter of 23G and an effective tube length of 1600 mm (Top endoscopic puncture needle "High Flow Type" for upper digestive tract: manufactured by TOP Corporation)

Endoscopic injection needle 2: Endoscopic puncture needle with a needle diameter of 23G and an effective tube length of 1650 mm (Disposable injection needle DNM NM-400L-0423: manufactured by Olympus Corporation)

Formulation examples of injection composition

Xanthan gum (KELTROL CGT manufactured by CP Kelco) (hereinafter, abbreviated as "XG") was dissolved in water for injection to prepare a 4 w/v% aqueous XG solution. The 4 w/v% aqueous XG solution was diluted with the water for injection to prepare 0.05 to 4 w/v% (concentration shown in Table 1) aqueous solutions. Each of the aqueous solutions was enclosed in a vial and subjected to autoclave treatment at 121°C for 20 minutes to prepare an injection composition. The viscosity (cP) and injection pressure (kgf) of each of the injection compositions and purified water were measured. The viscosity was measured at a liquid temperature of 25°C using a Viscomate vibration-type viscometer (VISCOMATE VM-1G manufactured by YAMAICHI ELECTRONICS CO., LTD.).

Relationship between viscosity and injection pressure of injection composition when endoscopic injection needle 1 was used

[Table 1]

| XG concentration (w/v%) | Viscosity (cP) | Injection pressure (kgf) | Difficulty of injection as determined manually |
|---|---|---|---|
| 0.05 | 1.80 | 0.5 | Easily injectable |
| 0.10 | 2.82 | 0.7 | Easily injectable |
| 0.20 | 5.24 | 1.0 | Easily injectable |

(continued)

| XG concentration (w/v%) | Viscosity (cP) | Injection pressure (kgf) | Difficulty of injection as determined manually |
|---|---|---|---|
| 0.25 | 6.97 | 1.3 | Easily injectable |
| 0.5 | 17.2 | 1.7 | Easily injectable |
| 1.0 | 50.1 | 2.6 | Easily injectable |
| 1.5 | 67.6 | 3.6 | Easily injectable |
| 2.0 | 72.7 | 4.8 | Easily injectable |
| 2.5 | 83.9 | 6.4 | Easily injectable |
| 3.0 | 109 | 8.3 | Slightly difficult but injectable |
| 3.5 | 197 | 10.9 | Difficult but injectable |
| 4.0 | 218 | 14.7 | Difficult to inject |
| 0 (water) | 0.89 | 0.36 | Easily injectable |

Relationship between viscosity and injection pressure of injection composition when endoscopic injection needle 2 was used

[Table 2]

| XG concentration (w/v%) | Viscosity (cP) | Injection pressure (kgf) | Difficulty of injection as determined manually |
|---|---|---|---|
| 0.05 | 1.80 | 1.1 | Easily injectable |
| 0.10 | 2.82 | 1.4 | Easily injectable |
| 0.20 | 5.24 | 2.1 | Easily injectable |
| 0.25 | 6.97 | 2.4 | Easily injectable |
| 0.5 | 17.2 | 3.3 | Easily injectable |
| 1.0 | 50.1 | 4.7 | Easily injectable |
| 1.5 | 67.6 | 6.6 | Easily injectable |
| 2.0 | 72.7 | 8.9 | Slightly difficult but injectable |
| 2.5 | 83.9 | 11.6 | Difficult but injectable |
| 3.0 | 109 | 14.7 | Difficult to inject |
| 3.5 | 197 | 19.8 | Difficult to inject |
| 4.0 | 218 | 21.4 | Difficult to inject |
| 0 (water) | 0.89 | 0.72 | Easily injectable |

As can be seen from the above experimental results, the injection pressure that can be used for practical use of XG is 1.0 to 12 kgf, preferably 1.0 to 8 kgf, and the XG concentration for practical use is 0.05 to 3.5 w/v%, preferably 0.1 to 3.0 w/v%, more preferably 0.2 to 2.5 w/v%.

Example 2

Performance of xanthan gum

Xanthan gum (KELTROL CGT manufactured by CP Kelco) (hereinafter, abbreviated as "XG") was dissolved in an water for injection to prepare a 1 w/v% aqueous solution. The 1 w/v% aqueous XG solution was diluted with the water for injection to prepare 0.2 to 1 w/v% (concentration shown in Table 3) aqueous solutions. Each of the aqueous solutions was enclosed in a vial and subjected to autoclave treatment at 121°C for 20 minutes to prepare an injection composition. On the other hand, a 1 w/v% sodium hyaluronate (hereinafter, sodium hyaluronate is abbreviated as "HA") injection composition (ARTZ joint injection 25 mg: manufactured by SEIKAGAKU CORPORATION) was diluted with a 0.9% aqueous sodium chloride solution to prepare 0.2 to 1 w/v% (concentration shown in Table 3) solutions as comparative solutions. The viscosity (cP) and injection pressure (kgf) of each of the injection compositions and comparative solutions were measured. The injection pressure (kgf) was measured by the method described in [0016]. However, as the endoscopic injection needle, an endoscopic puncture needle having a needle diameter of 23G and an effective tube length of 1650 mm (Disposable injection needle DNM NM-400L-0423 manufactured by Olympus Corporation) was used. The viscosity was measured at a liquid temperature of 25°C using a Viscomate vibration-type viscometer (VISCOMATE VM-1G manufactured by YAMAICHI ELECTRONICS CO., LTD.). The results are shown in Table 3.

[Table 3]

| XG or HA concentration (w/v%) | Viscosity (cP) | | Injection pressure (kgf) | |
|---|---|---|---|---|
| | XG | HA | XG | HA |
| 0.2 | 5.24 | 7.31 | 2.1 | 3.4 |
| 0.4 | 12.2 | 19.9 | 3.0 | 6.2 |
| 0.6 | 23.2 | 36.2 | 3.5 | 10.0 |
| 0.8 | 34.7 | 61.2 | 4.0 | 15.0 |
| 1.0 | 50.1 | 95.9 | 4.7 | 20 or higher |

The results shown in Table 3 were plotted on a graph (Fig. 2), wherein the vertical axis represents the injection pressure and the horizontal axis represents the viscosity.

As can be seen from Fig. 2, the XG injection composition having high viscosity at rest can be injected at a lower injection pressure as compared to the HA solution.

Example 3

Experiment for comparison of degree of mucosal lift

Xanthan gum (KELTROL CGT: manufactured by CP Kelco) (hereinafter, abbreviated as "XG") was dissolved in phosphate buffered saline (pH 7.4, osmotic pressure ratio 1) (hereinafter, abbreviated as "PBS") to prepare a 1 w/v% XG PBS solution. The 1 w/v% XG PBS solution was diluted with PBS to prepare 0.5 w/v% and 0.25 w/v% XG PBS solutions. Each of the XG PBS solutions different in XG concentration was enclosed in a vial and subjected to autoclave treatment at 121°C for 20 minutes to prepare an injection composition. The pH, osmotic pressure ratio, viscosity (cP), and injection pressure (kgf) of each of the injection compositions and a 0.4 w/v% HA solution (Mucoup: manufactured by SEIKAGAKU CORPORATION) were measured. The injection pressure (kgf) was measured by the method described in [0016]. However, as the endoscopic injection needle, an endoscopic puncture needle having a needle diameter of 23G and an effective tube length of 1650 mm (Disposable injection needle DNM NM-400L-0423 manufactured by Olympus Corporation) was used. The viscosity was measured at a liquid temperature of 25°C using a Viscomate vibration-type viscometer (VISCOMATE VM-1G manufactured by YAMAICHI ELECTRONICS CO., LTD.). The measurement results are shown in Table 4.

[Table 4]

| XG or HA concentration (w/v%) | pH | Osmotic pressure ratio | Viscosity (cP) | Injection pressure (kgf) |
|---|---|---|---|---|
| 0.25%XG | 6.71 | 1.05 | 3.84 | 2.4 |
| 0.5%XG | 6.42 | 1.07 | 12.6 | 3.7 |
| 1.0%XG | 6.09 | 0.99 | 39.0 | 5.9 |
| 0.4%HA | - | - | 16.7 | 6.1 |

Example 4

Experiment for comparison of degree of mucosal lift

An experiment for comparison of degree of mucosal lift was performed using the injection compositions described in Table 4 and PBS and the 0.4 w/v% HA solution (Mucoup: manufacture by SEIKAGAKU CORPORATION) as controls.

As test animals, 30 male Japanese white rabbits with a body weight of about 3 kg (available from KITAYAMA LABES CO., LTD.) were prepared. The rabbits were anesthetized by inhalation of isoflurane (manufactured by ABBOTT JAPAN CO., LTD. under the trade name of Forane) as an inhalation anesthetic and subjected to the experiment. After abdominal shaving, a midline incision was made in the abdominal wall of each of the rabbits to expose the stomach, and then 0.5 mL of each of the injection compositions was injected into the gastric submucosa from the gastric serosal side. The injection of the injection composition was performed at two sites on the stomach per rabbit, and the injection composition injected into the anterior wall of the stomach and the injection composition injected into the posterior wall of the stomach were different from each other. This experiment was repeated six times using the different test animals (n = 6). Just after or 30 minutes after the injection of the injection composition, each of the rabbits was killed by intravenous overdose of sodium thiopental (manufactured by Mitsubishi Tanabe Pharma Corporation under the trade name of Ravonal) and tissues of the injection sites were excised. The excised tissues were frozen in isopentane cooled at -80°C and vertically cut with a metal saw from the top of the injection site to measure the thickness of the gastric submucosa. The results are shown in Fig. 3. Further, from the results of measuring the submucosal thickness, the ratio of the thickness after 30 minutes of injection to the thickness just after injection was determined as a submucosal thickness retention ratio (%). The results are shown in Table 5.

[Table 5]

| XG or HA concentration (w/v%) | Submucosal thickness retention ratio (%) |
|---|---|
| 0% (PBS) | 72 |
| 0.25%XG | 89 |
| 0.5%XG | 90 |
| 1.0%XG | 99 |
| 0.4%HA | 81 |

$$\text{Submucosal thickness retention ratio (\%)} = \text{submucosal thickness (mm) after 30 minutes of injection} \div \text{submucosal thickness (mm) just after injection (mm)} \times 100$$

Just after injection, the 0.5% XG injection composition and the 0.4 w/v% HA solution were equally effective for mucosal lift, but after 30 minutes of injection, the 0.5% XG injection composition was more effective for mucosal lift. Further, XG is superior to HA in the ability to maintain mucosal lift for 30 minutes after injection, and therefore XG can be satisfactorily used as a composition for submucosal cushion agent even at a concentration of 0.25%.

Example 5

Experiment for comparison of degree of mucosal lift 2

The pH, osmotic pressure ratio, viscosity (cP), and injection pressure (kgf) of each of a 0.5 w/v% XG injection composition of Formulation Example 15 and a 0.4 w/v% HA solution (Mucoup manufactured by SEIKAGAKU CORPORATION) were measured. The injection pressure (kgf) was measured by the method described in [0016]. However, as the endoscopic injection needle, an endoscopic puncture needle having a needle diameter of 23G and an effective tube length of 1650 mm (Disposable injection needle DNM NM-400L-0423 manufactured by Olympus Corporation) was used. The viscosity was measured at a liquid temperature of 25°C using a Viscomate vibration-type viscometer (VISCOMATE VM-1G manufactured by YAMAICHI ELECTRONICS CO., LTD.). The measurement results are shown in Table 6.

[Table 6]

| XG or HA concentration (w/v%) | pH | Osmotic pressure ratio | Viscosity (cP) | Injection pressure (kgf) |
|---|---|---|---|---|
| 0.5%XG | 7.67 | 0.97 | 19.4 | 3.2 |
| 0.4%HA | - | - | 16.6 | 5.7 |

Example 6

Experiment for comparison of degree of mucosal lift 2

An experiment for comparison of degree of mucosal lift was performed using the 0.5 w/v% XG injection composition described in Table 6 and normal saline (manufactured by Otsuka Pharmaceutical Co., Ltd.) and a 0.4 w/v% HA solution as controls.

As test animals, 27 male Japanese white rabbits with a body weight of about 3 kg (available from KITAYAMA LABES CO., LTD.) were prepared. The rabbits were anesthetized by inhalation of isoflurane (manufactured by ABBOTT JAPAN CO., LTD. under the trade name of Forane) as an inhalation anesthetic and subjected to the experiment. After abdominal shaving, a midline incision was made in the abdominal wall of each of the rabbits to expose the stomach, and then 0.5 mL of each of the injection compositions was injected into the gastric submucosa from the gastric serosal side. The injection of the injection composition was performed at two sites on the stomach per rabbit, and the injection composition injected into the anterior wall of the stomach and the injection composition injected into the posterior wall of the stomach were different from each other. This experiment was repeated six times using the different test animals (n = 6). Just after or 30 minutes after the injection of the injection composition, each of the rabbits was killed by intravenous overdose of sodium thiopental (manufactured by Mitsubishi Tanabe Pharma Corporation under the trade name of Ravonal) and tissues of the injection sites were excised. The excised tissues were frozen in isopentane cooled at -80°C and vertically cut with a metal saw from the top of the injection site to measure the thickness of the gastric submucosa. The results are shown in Fig. 4. Further, from the results of measuring the submucosal thickness, the ratio of the thickness after 1 hour and 4 hours of injection to the thickness just after injection was determined as a submucosal thickness retention ratio (%). The results are shown in Table 7.

[Table 7]

| XG or HA concentration (w/v%) | Submucosal thickness retention ratio (%) | |
|---|---|---|
| | One hour after injection | Four hours after injection |
| Normal saline | 64 | 49 |
| 0.5 w/v% XG injection composition (Formulation Example 15) | 87 | 71 |
| 0.4 w/v% HA solution | 71 | 58 |

$$\text{Submucosal thickness retention ratio } (\%) = \text{Submucosal thickness}$$
$$\text{after one hour or four hours of injection (mm)} \div \text{Submucosal thickness just}$$
$$\text{after injection (mm)} \times 100$$

Just after injection, mucosal lift produced by the 0.5 w/v% XG injection composition was highest, followed by the 0.4 w/v% HA solution and the normal saline. Then, after 1 hour, the mucosal lift produced by the normal saline was reduced to about 2.1 mm and therefore could not be identified by visual observation of the excised tissue. From just after injection to after 4 hours of injection, the 0.5 w/v% XG injection composition was much superior to the 0.4 w/v% HA solution in the ability to maintain mucosal lift.

Example 7

Pseudoplastic viscosity

Xanthan gum (KELTROL CGT: manufactured by CP Kelco) (hereinafter, abbreviated as "XG") was dissolved in water for injection to prepare 1.0 and 0.5 w/v% aqueous XG solutions. Each of the aqueous solutions was enclosed in a vial and subjected to autoclave treatment at 121°C for 20 minutes to prepare 1.0 and 0.5 w/v% XG injection compositions. The viscosity of each of the injection compositions and a 0.4 w/v% HA solution (Mucoup: manufactured by SEIKAGAKU CORPORATION) as a comparative solution was measured using a digital viscometer (Models LVDV-II + Pro, spindle CP 52) manufactured by Brookfield Engineering Laboratories, Inc. at 25°C at different rotational speeds while the rotational speed was increased from an initial rotational speed of 1 rpm in increments of every 5 rpm, and the results are shown in Fig. 5.

Further, the viscosity of each of the 1.0 and 0.5 w/v% XG injection compositions and the 0.4 w/v% HA solution was measured using a digital viscometer (Models LVDV-II + Pro, spindle CP 52) manufactured by Brookfield Engineering Laboratories, Inc. at 25°C at a rotational speed of 1 rpm, then at 200 rpm, and then at 1 rpm. The measurement time at each rotational speed was set to 5 seconds, and the measurement was continuously performed.

The values of the viscosity (cP) at different rotational speeds (rpm) are shown in Fig. 6.

As shown in Fig. 5, the 1.0 and 0.5 w/v% injection compositions conspicuously exhibited pseudoplastic viscosity, that is, their viscosity tended to be extremely high in an initial state (i.e. in a near resting state) but significantly decrease with an increase in rotational speed (i.e. with an increase in applied force). However, the 0.4 w/v% HA solution did not exhibit pseudoplastic viscosity, that is, its viscosity did not have a tendency to be extremely high in an initial state.

Further, as shown in Fig. 6, in the case of the 1.0 and 0.5 w/v% injection compositions, a significant and instant (within a measurement time of 5 seconds) decrease in viscosity was observed with an increase in rotational speed, and then a significant and instantaneous (within a measurement time of 5 seconds) increase in viscosity (recovery of viscosity) was observed with a decrease in rotational speed. The 1.0 w/v% XG injection composition recovered about 80% of its initial viscosity, and the 0.5 w/v% XG injection composition recovered about 90% of its initial viscosity, that is, both the XG injection compositions could satisfactorily and instantaneously recover their viscosity. An attempt was made to perform the same measurement on a 1.0 w/v% HA solution, but the measurement could not be performed because after the measurement of viscosity at 25° at 1 rpm, the rotational speed could not be increased to 200 rpm due to high viscosity.

The above results indicate that the use of an aqueous solution having pseudoplastic viscosity makes it possible to achieve the object of the present invention, that is, to provide an excellent injection composition that can be injected at a low injection pressure (i.e. by the application of a weak force) but again has high viscosity in an injection site.

Example 8

Measurement of Casson yield value

Xanthan gum (KELTROL CGT: manufactured by CP Kelco) (hereinafter, abbreviated as "XG") was dissolved in water for injection to prepare a 4 w/v% aqueous XG solution. The 4 w/v% aqueous XG solution was diluted with the water for injection to prepare 0.05 to 2.0 w/v% (concentration shown in Table 8) aqueous solutions. Each of the aqueous solutions was enclosed in a vial and subjected to autoclave treatment at 121°C for 20 minutes to prepare an injection composition. A 0.5% aqueous locust bean gum solution (referred to as "0.5% LBG") of Formulation Example 10, an aqueous solution containing 0.25% xanthan gum and 0.25% locust beam gum (referred to as "0.25% XG + 0.25% LBG") of Formulation Example 13, and an aqueous solution containing 0.25% xanthan gum and 0.25% guar gum (referred to as "0.25% XG + 0.25% GG") of Formulation Example 14 were prepared. The Casson yield value of each of the injection compositions, Formulation Examples, and purified water was determined by measuring viscosity using a digital viscometer (Model: LVDV-II + Pro) manufactured by Brookfield Engineering Laboratories Inc. at 25°C at predetermined different rotational speeds, respectively. It is to be noted that a spindle CP40 was used for the measurement of viscosity of 0.05 to 0.25% XG, 0.2 and 0.4% HA, and purified water, and a spindle CP52 was used for the measurement of viscosity of 0.5 to 2.0% XG and Formulation Examples 10, 13, and 14. A formula for calculating the Casson yield value is shown in Fig. 1.

The results are shown in Table 8.

[Table 8]

| Formulation (w/v%) | Casson yield value (Unit: Dynes/$cm^2$) (Spindle No.) |
|---|---|
| 0.05%XG | 0.08 (CP40) |

(continued)

| Formulation (w/v%) | Casson yield value (Unit: Dynes/cm$^2$) (Spindle No.) |
|---|---|
| 0.1%XG | 0.12 (CP40) |
| 0.15%XG | 0.68 (CP40) |
| 0.2%XG | 0.73 (CP40) |
| 0.25%XG | 1.22 (CP40) |
| 0.5%XG | 20.3 (CP52) |
| 1.0%XG | 46.4 (CP52) |
| 2.0%XG | 57.8 (CP52) |
| 0.5% LBG (Formulation Example 10) | 3.26 (CP52) |
| 0.25% XG + 0.25% LBG (Formulation Example 13) | 66.5 (CP52) |
| 0.25% XG + 0.25% GG (Formulation Example 14) | 14.3 (CP52) |
| 0.2%HA | 0.0.2 (CP40) |
| 0.4%HA | 0.00 (CP40) |
| Purified water | 0.00 (CP40) |

Example 9

Formulation Example 1

| | |
|---|---|
| Xanthan gum | 1.0% |
| Water for injection | sufficient quantity (s. q.) |
| Total | 100.0% |

Xanthan gum (KELTROL CG-SFT) of 1 g was dissolved in 90 mL of water for injection, and then the water for injection was added so that the total volume of the solution was 100 mL. The obtained solution was filtered through a filter with a pore size of 5 $\mu$m, filled and sealed in a vial, and then subjected to high-pressure steam sterilization at 121°C for 20 minutes to obtain an injection composition. The injection composition was a colorless and clear liquid having a viscosity of 21.4 cP. The injection pressure of the injection composition was 4.7 kgf. The viscosity of the injection composition after storage at 60°C for 3 weeks was 20.6 cP.

Formulation Example 2

| | |
|---|---|
| Xanthan gum | 1.0% |
| Water for injection | s.q. |
| Total | 100.0% |

Xanthan gum (KELTROL CG-T) of 1 g was dissolved in 90 mL of water for injection, and then the water for injection was added so that the total volume of the solution was 100 mL. The obtained solution was filtered through a filter with a pore size of 5 $\mu$m, filled and sealed in a vial, and then subjected to high-pressure steam sterilization at 121°C for 20 minutes to obtain an injection composition. The injection composition was a colorless and clear liquid having a viscosity of 52.9 cP. The injection pressure of the injection composition was 5.0 kgf. The viscosity of the injection composition after storage at 60°C for 3 weeks was 44.1 cP.

Formulation Example 3

| | |
|---|---|
| Xanthan gum | 1.0% |
| Glycerin | 2.5% |
| Water for injection | s.q. |
| Total | 100.0% |

Xanthan gum (KELTROL CG-SFT) of 1 g was dissolved in 80 mL of water for injection, 2.5 g of glycerin was added to and dissolved in the solution, and the water for injection was added so that the total volume of the solution was 100 mL. The obtained solution was filtered with a filter with a pore size of 5 $\mu$m, filled and sealed in a vial, and subjected to high-pressure steam sterilization at 121°C for 20 minutes to obtain an injection composition. The injection composition was a colorless and clear liquid having a viscosity of 42.4 cP. The injection pressure of the injection composition was 5.1 kgf. The viscosity of the injection composition after storage at 60°C for 3 weeks was 37.3 cP.

Formulation Example 4

| | |
|---|---|
| Xanthan gum | 1.0% |
| Sodium chloride | 0.9% |
| Water for injection | s.q. |
| Total | 100.0% |

Xanthan gum (KELTROL CG-SFT) of 1 g was dissolved in 80 mL of water for injection, 0.9 g of sodium chloride was added to and dissolved in the solution, and the water for injection was added so that the total volume of the solution was 100 mL. The obtained solution was filtered with a filter with a pore size of 5 $\mu$m, filled and sealed in a vial, and then subjected to high-pressure steam sterilization at 121°C for 20 minutes to obtain an injection composition. The injection composition was a colorless and clear liquid having a viscosity of 26.1 cP. The injection pressure of the injection composition was 5.3 kgf. The viscosity of the injection composition after storage at 60°C for 3 weeks was 23.2 cP.

Formulation Example 5

| | |
|---|---|
| Xanthan gum | 1.0% |
| Mannitol | 2.5% |
| Water for injection | s.q. |
| Total | 100.0% |

Xanthan gum (KELTROL CG-SFT) of 1 g was dissolved in 80 mL of a water for injection, 2.5 g of mannitol was added to and dissolved in the solution, and the water for injection was added so that the total volume of the solution was 100 mL. The obtained solution was filtered with a filter with a pore size of 5 $\mu$m, filled and sealed in a vial, and then subjected to high-pressure steam sterilization at 121°C for 20 minutes to obtain an injection composition. The injection composition was a colorless and clear liquid having a viscosity of 42.1 cP. The injection pressure of the injection composition was 5.0 kgf. The viscosity of the injection composition after storage at 60°C for 3 weeks was 35.2 cP.

Formulation Example 6

| | |
|---|---|
| Xanthan gum | 1.0% |
| Maltose | 5.0% |
| Water for injection | s.q. |

(continued)

| | |
|---|---|
| Total | 100.0% |

Xanthan gum (KELTROL CG-SFT) of 1 g was dissolved in 80 mL of a water for injection, 5 g of maltose was added to and dissolved in the solution, and the water for injection was added so that the total volume of the solution was 100 mL. The obtained solution was filtered with a filter with a pore size of 5 μm, filled and sealed in a vial, and subjected to high-pressure steam sterilization at 121°C for 20 minutes to obtain an injection composition. The injection composition was a slightly yellowish and clear liquid having a viscosity of 38.4 cP. The injection pressure of the injection composition was 5.0 kgf. The viscosity of the injection composition after storage at 60°C for 3 weeks was 29.5 cP.

Formulation Example 7

| | |
|---|---|
| Xanthan gum | 1.0% |
| Glucose | 5.0% |
| Water for injection | s.q. |
| Total | 100.0% |

Xanthan gum (KELTROL CG-SFT) of 1 g was dissolved in 80 mL of water for injection, 5 g of mannitol was added to and dissolved in the solution, and the water for injection was added so that the total volume of the solution was 100 mL. The obtained solution was filtered through a filter with a pore size of 5 μm, filled and sealed in a vial, and then subjected to high-pressure steam sterilization at 121°C for 20 minutes to obtain an injection composition. The injection composition was a slightly yellowish and clear liquid having a viscosity of 41.3 cP. The injection pressure of the injection composition was 5.1 kgf. The viscosity of the injection composition after storage at 60°C for 3 weeks was 30.2 cP.

Formulation Example 8

| | |
|---|---|
| Xanthan gum | 0.8% |
| D-sorbitol | 5.0% |
| pH adjusting agent | s.q. |
| Water for injection | s.q. |
| Total | 100.0% (pH 7.7) |

Xanthan gum (KELTROL CG-T) of 0.8 g was dissolved in 80 mL of water for injection, 5 g of D-sorbitol was added to and dissolved in the solution, an appropriate amount of hydrochloric acid and Trometamol were added to adjust pH, and the water for injection was added so that the total volume of the solution was 100 mL. The obtained solution was filtered through a filter with a pore size of 5 μm, filled and sealed in a vial, and then subjected to high-pressure steam sterilization at 121°C for 20 minutes to obtain an injection composition. The injection composition was a colorless and clear liquid having a viscosity of 35.0 cP. The injection pressure of the injection composition was 4.8 kgf. The viscosity of the injection composition after storage at 60°C for 3 weeks was 33.4 cP.

Formulation Example 9

| | |
|---|---|
| Xanthan gum | 0.6% |
| D-sorbitol | 5.0% |
| pH adjusting agent | s.q. |
| Water for injection | s.q. |
| Total | 100.0% (pH 6.0) |

Xanthan gum (KELTROL CG-T) of 0.6 g was dissolved in 80 mL of an water for injection, 5 g of D-sorbitol was added to and dissolved in the solution, an appropriate amount of citric acid and sodium citrate were added to adjust pH, and the water for injection was added so that the total volume of the solution was 100 mL. The obtained solution was filtered through a filter with a pore size of 5 $\mu$m, filled and sealed in a vial, and then subjected to high-pressure steam sterilization at 121°C for 20 minutes to obtain an injection composition. The injection composition was a colorless and clear liquid having a viscosity of 24.2 cP. The injection pressure of the injection composition was 4.3 kgf. The viscosity of the injection composition after storage at 60°C for 3 weeks was 25.0 cP.

Formulation Example 10

| | |
|---|---|
| Purified locust bean gum | 0.5% |
| Water for injection | s.q. |
| Total | 100.0% |

Purified locust bean gum (GENUGUM type RL-200Z) of 0.5 g was added to and dissolved in 90 mL of water for injection at 80°C with stirring, and the water for injection was added so that the total volume of the solution was 100 mL. The obtained solution was filled and sealed in a vial and then subjected to high-pressure steam sterilization at 121°C for 20 minutes to obtain an injection composition. The injection composition was a colorless and clear liquid having a viscosity of 49.5 cP. The injection pressure of the injection composition was 8.2 kgf.

Formulation Example 11

| | |
|---|---|
| Gellan gum | 0.2% |
| Water for injection | s.q. |
| Total | 100.0% |

Gellan gum (KELCOGEL CG-HA) of 0.2 g was added to and dissolved in 90 mL of water for injection at 90°C with stirring, and the water for injection was added so that the total volume of the solution was 100 mL. The obtained solution was filled and sealed in a vial and then subjected to high-pressure steam sterilization at 121°C for 20 minutes to obtain an injection composition. The injection composition was a milky white liquid having a viscosity of 8.23 cP. The injection pressure of the injection composition was 2.9 kgf.

Formulation Example 12

| | |
|---|---|
| Gellan gum | 0.4% |
| Water for injection | s.q. |
| Total | 100.0% |

Gellan gum (KELCOGEL CG-HA) of 0.4 g was added to and dissolved in 90 mL of water for injection at 90°C with stirring, and the water for injection was added so that the total volume of the solution was 100 mL. The obtained solution was filled and sealed in a vial and subjected to high-pressure steam sterilization at 121°C for 20 minutes to obtain an injection composition. The injection composition was a milky white soft gel-like material having a viscosity of 30.0 cP. The injection pressure of the injection composition was 6.4 kgf.

Formulation Example 13

| | |
|---|---|
| Purified locust bean gum | 0.25% |

(continued)

| | |
|---|---|
| Xanthan gum | 0.25% |
| Water for injection | s.q. |
| Total | 100.0% |

A 0.5% aqueous locust bean gum (GENUGUM type RL-200Z) solution and a 0.5% aqueous xanthan gum (KELTROL CG-T) solution were mixed in a ratio of 1:1. The obtained mixed solution was filled and sealed in a vial and then subjected to high-pressure steam sterilization at 121°C for 20 minutes to obtain an injection composition. The injection composition was a colorless and clear liquid having a viscosity of 36.4 cP. The injection pressure of the injection composition was 6.3 kgf.

Formulation Example 14

| | |
|---|---|
| Guar gum | 0.25% |
| Xanthan gum | 0.25% |
| Water for injection | s.q. |
| Total | 100.0% |

Guar gum (Supergel CSA200/50) of 0.5 g was added to 90 mL of water for injection with stirring, and the water for injection was added so that the total volume of the solution was 100 mL. The obtained solution and a 0.5% aqueous xanthan gum (KELTROL CG-T) solution were mixed in a ratio of 1 : 1, and the mixed solution was filled and sealed in a vial and subjected to high-pressure steam sterilization at 121°C for 20 minutes to obtain an injection composition. The injection composition was a slightly milky white liquid having a viscosity of 18.1 cP. The injection pressure of the injection composition was 4.5 kgf.

Example 10

Formulation Example 15

| | |
|---|---|
| Xanthan gum | 0.5% |
| D-sorbitol | 1.0% |
| Sodium chloride | 0.45% |
| pH adjusting agent | s.q. |
| Water for injection | s.q. |
| Total | 100.0% (pH 7.7) |

Xanthan gum (KELTROL CG-T) of 1.5 g was dissolved in 240 mL of an water for injection, 3 g of D-sorbitol and 1.35 g of sodium chloride were added to and dissolved in the solution, an appropriate amount of hydrochloric acid and trishydroxymethyl-aminomethane (Tris) were added to adjust pH, and the water for injection was added so that the total volume of the solution was 300 mL. The obtained solution was filtered through a filter with a pore size of 5 $\mu$m, filled and sealed in a vial, and then subjected to high-pressure steam sterilization at 121°C for 20 minutes to obtain an injection composition. The injection composition was a colorless and clear liquid having a viscosity of 19.4 cP. The injection pressure of the injection composition was 3.2 kgf.

INDUSTRIAL APPLICABILITY

The present invention relates to a material used to lift mucosal tissue during endoscopic resection of the mucosal tissue, and also relates to use of a polysaccharide having pseudoplastic flow properties, such as xanthan gum, as a material.

The use of such a polysaccharide having pseudoplastic flow properties makes it possible to obtain an injection composition that is cheaper and safer, can maintain lift without diffusing even when injected into a lesion site, and can be injected at a low injection pressure and is therefore excellent in handle ability.

DESCRIPTION OF REFERENCE SIGNS

The symbols in Fig. 1 have the following meanings, respectively.

$\tau$: shear stress
$\tau_0$: Casson yield value (Dynes/cm$^2$)
$\eta$: plastic viscosity (cP)
D: shear rate

The symbols $\Delta$ and $\square$ in Fig. 2 correspond to xanthan gum and sodium hyaluronate, respectively.
In the bar graph shown in Fig. 3, the left-side (dotted) bar represents the thickness of gastric submucosa just after injection and the right-side (shaded) bar represents the thickness of gastric submucosa 30 minutes after injection.
In the bar graph shown in Fig. 4, the left-side (white) bar, the middle (dotted) bar, and the right-side (shaded) bar represent the thickness of gastric submucosa just after injection, the thickness of gastric submucosa 1 hour after injection, and the thickness of gastric submucosa 4 hours after injection , respectively.
The symbols $\square$, $\Delta$, and $\bigcirc$ in Fig. 5 correspond to 1.0% XG, 0.5% XG, and sodium hyaluronate, respectively.
In the bar graph shown in Fig. 6, the left-side (dotted) bar, the middle (black) bar, and the right-side (shaded) bar represent viscosity measured at an initial rotational speed of 1 rpm, then at 200 rpm, and then at 1 rpm, respectively.

**Claims**

1. A composition for submucosal cushion agent comprising a polysaccharide having pseudoplastic viscosity.

2. The composition for submucosal cushion agent according to claim 1, wherein the polysaccharide having pseudoplastic viscosity is xanthan gum, carrageenan, gellan gum, guar gum, locust bean gum, sacran, or a salt thereof.

3. The composition for submucosal cushion agent according to claim 1 or 2, wherein the polysaccharide having pseudoplastic viscosity is xanthan gum or a salt thereof.

4. The composition for submucosal cushion agent according to any one of claims 1 to 3, wherein the polysaccharide has a concentration of 0.05 to 3.5 w/v%.

5. The composition for submucosal cushion agent according to any one of claims 1 to 4, wherein an injection pressure of the composition, when pushed out of an endoscopic injection needle having a needle diameter of 23 G connected to a tube with an effective length of 1600 mm measured by a texture analyzer (EZ Test 500 N: manufactured by SHIMADZU CORPORATION) at a constant rate of 100 mm/min, is 0.1 to 12 kgf.

6. A method of forming a submucosal cushion in a subject which comprises injecting the polysaccharide having pseudoplastic viscosity described in any one of the above (1) to (5) in a submucosa of the subject.

[Fig. 1]

$$\sqrt{\tau} = \sqrt{\tau_0} + \sqrt{nD}$$

[Fig. 2]

[Fig. 3]

[Fig. 4]

[Fig. 5]

[Fig. 6]

## INTERNATIONAL SEARCH REPORT

International application No.

PCT/JP2012/080172

### A. CLASSIFICATION OF SUBJECT MATTER
*A61L31/00*(2006.01)i

According to International Patent Classification (IPC) or to both national classification and IPC

### B. FIELDS SEARCHED

Minimum documentation searched (classification system followed by classification symbols)
A61L31/00

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)
JSTPlus/JMEDPlus(JDreamII), CA/MEDLINE/EMBASE/BIOSIS(STN)

### C. DOCUMENTS CONSIDERED TO BE RELEVANT

| Category* | Citation of document, with indication, where appropriate, of the relevant passages | Relevant to claim No. |
|---|---|---|
| X/A | JP 2001-192336 A  (Hironori YAMAMOTO),<br>17 July 2001 (17.07.2001),<br>claims 1 to 5; paragraphs [0013] to [0019]<br>& US 6319260 B1 | 1,4,5/2,3 |
| A | JP 2004-513872 A  (Genentech, Inc.),<br>13 May 2004 (13.05.2004),<br>paragraph [0012]<br>& JP 4871470 B          & US 7582311 B1<br>& US 2007/0098736 A1     & US 2010/0330184 A1<br>& EP 1220690 A          & WO 2001/028591 A2<br>& AU 1071201 A          & CA 2387058 A | 1-5 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07 January, 2013 (07.01.13) | 22 January, 2013 (22.01.13) |

| Name and mailing address of the ISA/<br>Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2012/080172

C (Continuation).    DOCUMENTS CONSIDERED TO BE RELEVANT

| Category* | Citation of document, with indication, where appropriate, of the relevant passages | Relevant to claim No. |
|---|---|---|
| X/A | Masaaki TAJIMA et al., "Naishikyoteki Nenmaku Kaso Hakurijutsu ni Okeru Atarashii Nenmakuka Chunyu Busshitsu: Sodium Alginate no Kaihatsu", Gastroenterol Endosc, 15 September 2007 (15.09. 2007), vol.49, no.Supplement 2, page 2295, (Nai P-141) | 1,4/2,3,5 |
| A | Masaya HIROHATA, "Tennen-kei Tatorui Oyobi sono Yudotai no Keshohin eno Oyo", FRAGRANCE JOURNAL, vol.33, no.3, 2005.03, 19 to 29 | 1-5 |

Form PCT/ISA/210 (continuation of second sheet) (July 2009)

**INTERNATIONAL SEARCH REPORT**

International application No.

PCT/JP2012/080172

**Box No. II      Observations where certain claims were found unsearchable (Continuation of item 2 of first sheet)**

This international search report has not been established in respect of certain claims under Article 17(2)(a) for the following reasons:

1. ☒ Claims Nos.:  6
        because they relate to subject matter not required to be searched by this Authority, namely:
        Claims 6 pertains to method for treatment of the human body or animal body
by surgery or therapy and thus relates to a subject matter on which this
International Searching Authority is not required to carry out an international
search under the provisions of PCT Article 17(2)(a)(i) and [PCT Rule 39.1(iv)].

2. ☐ Claims Nos.:
        because they relate to parts of the international application that do not comply with the prescribed requirements to such an
        extent that no meaningful international search can be carried out, specifically:

3. ☐ Claims Nos.:
        because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable
        claims.

2. ☐ As all searchable claims could be searched without effort justifying additional fees, this Authority did not invite payment of
        additional fees.

3. ☐ As only some of the required additional search fees were timely paid by the applicant, this international search report covers
        only those claims for which fees were paid, specifically claims Nos.:

4. ☐ No required additional search fees were timely paid by the applicant.   Consequently, this international search report is
        restricted to the invention first mentioned in the claims; it is covered by claims Nos.:

**Remark on Protest**        ☐ The additional search fees were accompanied by the applicant's protest and, where applicable, the
                                         payment of a protest fee.

                                      ☐ The additional search fees were accompanied by the applicant's protest but the applicable protest
                                         fee was not paid within the time limit specified in the invitation.

                                      ☐ No protest accompanied the payment of additional search fees.

Form PCT/ISA/210 (continuation of first sheet (2)) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000037240 A
- JP 4176475 B
- JP 4761921 B

**Non-patent literature cited in the description**

- *Gastrointest. Endosc.,* 1999, vol. 50 (5), 701-704
- *Gastrointest. Endosc.,* 1999, vol. 50 (2), 251-256